# EUROPEAN PATENT APPLICATION

(11) **EP 0 682 113 A2**
(43) Date of publication of application: **15.11.1995**
(21) Application number: 95106929.3
(22) Date of filing: 08.05.1995
(51) Int. Cl.: C12N 15/12, C07K 14/705, C12N 15/85, C12N 5/10, C07K 16/28, A61K 38/22

(54) **Polypeptide produced in an endothelial cell line and DNA encoding it**

(30) Priority: 12.05.1994 JP 123155/94
(71) Applicant: ONO PHARMACEUTICAL CO., LTD., Osaka-shi Osaka (JP)
(72) Inventor: Shibayama, Shiro, c/o Minase Research Institute, Shimamoto-cho, Mishima-gun, Osaka (JP); Hirano, Atsushi, c/o Minase Research Institute, Shimamoto-cho, Mishima-gun, Osaka (JP); Ohno, Hiroyuki, c/o Minase Research Institute, Shimamoto-cho, Mishima-gun, Osaka (JP)
(74) Representative: Henkel, Feiler, Hänzel & Partner

(57) **Abstract**

The present invention is related to novel polypeptide consisting of 205 amino acids which is produced in an endothelial cell line of human umbilical cord vein, a method of producing it, DNA encoding the polypeptide, a vector comprising the DNA, host cells transformed or transfected with the vector, an antibody to the polypeptide and pharmaceutical composition containing the polypeptide or antibody. The polypeptide of the present invention may be useful as itself or secretion type for the prevention of or in treatment of immune diseases caused by adhesion of platelet, many kind of leukocytes and macrophage and disease relating to thrombus formation (e.g. rheumatoid arthritic, allergy, arteriosclerosis, rejection after an internal organ plantation, myocardial infarction, brain infraction or reperfusion failure caused by it, DIC and septicemia etc.), or for screening test of the prevention of and/or in treatment of the above disease.

## Description

### Field of the Invention

The present invention is related to a novel polypeptide produced by a certain vascular endothelial cell, a method of producing it, DNAs encoding the polypeptide, a vector comprising the DNA, host cells transformed or transfected with the vector, an antibody to the polypeptide and a pharmaceutical composition containing the polypeptide or the antibody.

### Background of the Invention

Vascular organization consists of endothelial cells, smooth muscle cells and fibroblasts. Especially, endothelial cells have the following serious functions;
1) regulation of vasotonia,
2) regulation of antithrombus and
3) response to disturbance factors of inflammation or immunity.
It is thought that disturbance of the endothelial cells or anomaly of functional modulation is one of causes of arteriosclerosis or inflammatory diseases.

### Related Arts

On endothelial cells, adhesion molecules (e.g. VCAM-1, ICAM-1, E-selectin and P-selectin) which have adhesive action to platelet, leukocyte or macrophage, and MAdCAM and GlyCAM which are ligands of adhesion molecule e.g. L-selectin, are constantly produced. When inflammation occurs, many kinds of cytokines are produced on the surface of the cells by many kinds of stimuli.

On the other hand, substances ( e.g. endothelin and angiotensin II etc.) which result in constriction and relaxation of blood vessel by stimulating of smooth muscle cells are produced. Further, substances which keep antithrombosis, e.g., thrombomodulin which anti-activates thrombin and activates protein C, plasminogen activator which enhances blood hyperfibrinolysis, and proteoglycans which has heparan sulfate prevents adhesion of platelet are produced. Amino acid sequences of most of these factors have been revealed. At present, they have been treated as a subject of not only pure research but also study and development for the purpose of applying to pharmaceuticals.

### Purpose of the Invention

As described previously, it is cleared that factors concerning regulation of vasotonia, response to disturbance factors of inflammation or immunity and regulation of antithrombus are produced from vascular endothelial cells. These facts suggest that the other factors which have the same functions are produced from endothelial cells.

The present inventors have directed their attention to this point and energetic research has been carried out in order to find novel factors which a endothelial cell generates.

Until now, when a man skilled in the art intends to obtain a particular polypeptide or a DNA encoding it, he generally utilizes methods by confirming an intended biological activity in a tissue or in a cell medium, isolating and purifying the polypeptide and then cloning a gene or methods by "expression-cloning" with the guidance of the biological activity.

However, physiologically active polypeptides in living body have often many kinds of activities. Therefore, it is increasing that after a gene is cloned, the gene is found to be identical to that encoding a polypeptide already known. Generally cell generates only a very slight amount of a factor and it makes difficult to isolate and to purify the factor and to confirm its biological activity.

On the other hand, preparation technique and sequencing technique of cDNA have been rapidly developed, and it has been able to sequence a large quantity of cDNA. Further, methods of "Reverse Genetics" of characterizing the function of a gene from the sequence of the gene, have been greatly developed.

The present inventors attempted to find novel polypeptides by using these methods. That is, a series of methods was carried out by taking out gene from vascular endothelial cell etc. and isolating mRNA, obtaining cDNA by using mRNA thus obtained as a starting material, deciding its nucleotide sequence and deducing its amino acid sequence. In this manner, the present inventors have succeeded to find a novel membrane protein and DNAs encoding it to complete the present invention.

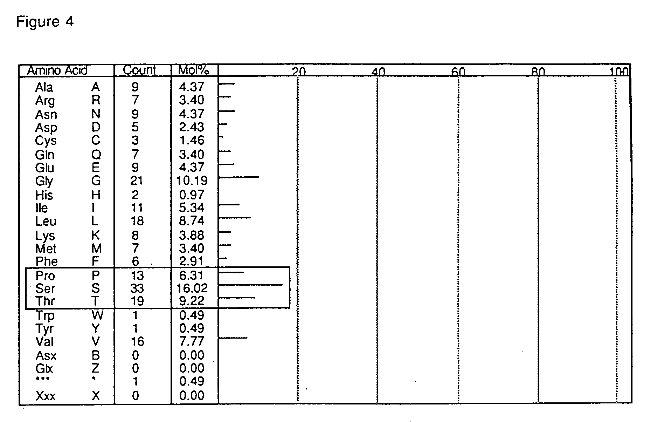

It is confirmed that the polypeptide of the present invention have the feature as membrane protein. The amino acid constitution of the polypeptide is shown in Figure 4. The polypeptide is confirmed to have the character of adhesion molecule because it includes a lot of Ser, Thr and Pro.

### Constitution of the Invention

The present invention is related to:
(1) a polypeptide having an amino acid sequence shown in SEQ ID No. 1,
(2) a DNA encoding the polypeptide described above (1),
(3) a DNA having a nucleotide sequence shown in SEQ ID No. 2, and
(4) a DNA having a nucleotide sequence shown in SEQ ID No. 3.

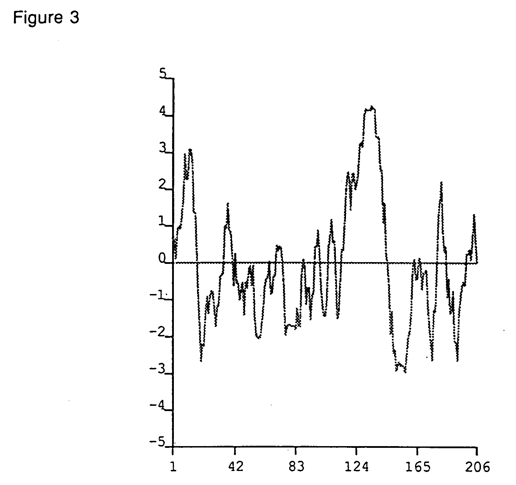

It is known that most cytokines and growth factors, as well as a number of membrane proteins, have signal peptides on their N-termini. The signal peptide is a highly hydrophobic region immediately down stream of the translation initiation amino acid Met. It was confirmed that the polypeptide of the present invention has a signal peptide region which is located from serine (Ser) at the 1st position to glycine (Gln) at the 24th position in the amino acid sequence shown in SEQ ID No.1. The essential sequence for biological activity is the amino acid sequence where the signal peptide is removed from the amino acid sequence. The signal peptide is not required for biological activity. On the other hand, the peptide of the present invention was confirmed transmembrane domain which is located from amino acid at the 119th position to amino acid at the 146th position in the amino acid sequence shown by SEQ ID No. 1 (see Figure 3).

The present invention is concerned with a polypeptide having the amino acid shown in SEQ ID No. 1, in substantially purified form, a homologue thereof, a fragment of the sequence and homologue of a fragment, and DNA encoding such a polypeptide. More particularly, the present invention is related to DNA having the nucleotide sequence shown in SEQ ID No. 2 or 3, and DNA having a fragment which is selectively hybridizing to nucleotide sequence shown in SEQ ID No. 2 or 3.

A polypeptide of SEQ ID No. 1 in substantially purified form will generally comprise the polypeptide in a production in which more than 90%, e.g. 95%, 98% or 99% of the polypeptide in the production is that of the SEQ ID No. 1.

A polypeptide homologue of the SEQ ID No. 1 will be generally at least 70%, preferably at least 80 or 90% and more preferably at least 95% homologous to the polypeptide of SEQ ID No. 1 over a region of at least 5, preferably at least 10, for instance 15, 20 or 25 more contiguous amino acids. Such polypeptide homologues will be referred to below as a polypeptide according to the invention.

Generally, fragments of SEQ ID No. 1 or its homologues will be at least 5, preferably at least 10, for example 15, 20 or 25 amino acids in length, and are also encompassed by the term "a polypeptide according to the invention" as used herein.

A DNA capable of selectively hybridizing to the DNA of SEQ ID No. 2 or 3 will be generally at least 70%, preferably at least 80 or 90% and more preferably at least 95% homologous to the DNA of SEQ ID No. 2 or 3 over a region of at least 20, preferably at least 30, for instance 40, 60 or 100 or more contiguous nucleotides. Such DNA will be encompassed by the term "DNA according to the invention".

Fragments of the DNA of SEQ ID No. 2 or 3 will be at least 10, preferably at least 15, for example 20, 25, 30 or 40 nucleotides in length, and are also encompassed by the term "DNA according to the invention" as used herein.

A further embodiment of the invention provides replication and expression vectors comprising DNA according to the invention. The vectors may be, for example, plasmid, virus or phage vectors provided with an origin of replication, optionally a promoter for the expression of the said DNA and optionally a regulator of the promoter. The vector may contain one or more selectable marker genes, for example a anpicillin resistance gene. The vector may be used in vitro, for example of the production of RNA corresponding to the DNA, or used to transfect or transform a host cell.

A further embodiment of the invention provides host cells transformed or transfected with the vectors for the replication and expression of DNA according to the invention, including the DNA SEQ. ID No. 2 or 3 or the open reading frame thereof. The cells will be chosen to be compatible with the vector and may for example be bacterial, yeast, insect or mammalian.

A further embodiment of the invention provides a method of producing a polypeptide which comprises culturing host cells of the present invention under conditions effective to express a polypeptide of the invention. Preferably, in addition, such a method is carried out under conditions in which the polypeptide of the invention is expressed and then produced from the host cells.

DNA according to the invention may also be inserted into the vectors described above in an antisense orientation in order to proved for the production of antisense RNA. Antisense RNA may also be produced by synthetic means. Such antisense RNA may be used in a method of controlling the levels of a polypeptide of the invention in a cell.

The invention also provides monoclonal or polyclonal antibodies to a polypeptide according to the invention. The invention further provides a process for the production of monoclonal or polyclonal antibodies to the polypeptides of the invention. Monoclonal antibodies may be prepared by conventional hybridoma technology using a polypeptide of the invention or a fragment thereof, as an immunogen. Polyclonal antibodies may also be prepared by conventional means which comprise inoculating a host animal, for example a rat or a rabbit, with a polypeptide of the invention and recovering immune serum.

The present invention also provides pharmaceutical compositions containing a polypeptide of the invention, or an antibody thereof, in association with a pharmaceutically acceptable diluent and/or carrier.

The polypeptide of the present invention includes that which a part of their amino acid sequence is lacking (e.g., a polypeptide comprised of the only essential sequence for revealing a biological activity and a soluble polypeptide lacking cytoplasmic domains and transmembrane domains in an amino acid sequence shown in SEQ ID No.1), that which a part of their amino acid sequence is replaced by other amino acids (e.g., those replaced by an amino acid having a similar property) and that which other amino acids are added or inserted into a part of their amino acid sequence, as well as those having the amino acid sequence shown in SEQ ID No. 1.

As known well, there are one to six kinds of codon as that encoding one amino acid (for example, one kind of codon for Met, and six kinds of codon for Leu) are known. Accordingly, the nucleotide sequence of DNA can be changed in order to encode the polypeptide having the same amino acid sequence.

The DNA of the present invention, specified in (2) includes a group of every nucleotide sequences encoding polypeptides (1) shown in SEQ ID No. 1 . There is a probability of improving a yield of production of a polypeptide by changing a nucleotide sequence.

The DNA of SEQ ID No. 2 specified in (3) is the embodiment of DNA shown in (2), and is sequence in the natural form.

The DNA of SEQ ID No. 3 shown in (4) indicates the sequence of the DNA specified in (3) with a non-translational region.

The DNA having a nucleotide sequence shown in SEQ ID No. 3 may be prepared according to the following methods, that is:
(i) by isolating mRNA from a cell line which produces the polypeptide of the present invention (e.g., human vascular endothelial cell line),
(ii) by preparing first strand (single stranded DNA) from mRNA thus obtained, followed by preparing second strand (double stranded DNA),
(iii) by inserting cDNA thus obtained into a proper plasmid vector,
(iv) by transforming host cells with the recombinant DNA thus obtained,
(v) by random-cloning on a large scale from cDNA library thus obtained, followed by sequencing average 300 bases from 5' end of each clone, and
(vi) by sequencing complete length of a clone which has a novel base sequence.

Explained in detail, step (i) may be carried out by using human vascular endothelial cell line in accordance with the method of Okayama, H. et al. (described in Methods in Enzymology, vol. 154, pp 3, 1987). Examples of the cells which produce the polypeptide of the present invention is preferably endothelial cell line of human umbilical cord vein. Steps (ii), (iii) and (iv) are a series of steps for preparing cDNA library, and may be carried out in accordance with the method of Gubler & Hoffman (Gene, vol. 25, pp. 263, 1983) with a slight modification. As examples of the plasmid vector used in the step (iii), many vectors functioning in an *E. coli* strain (e.g., pBR 322) and in a Bacillus subtilis (e.g., pUB 110) are known, and pUCSRαML-1 (described in detail in the following Example) prepared from pUC19 and pcD-SRα296 which functions in an *E. coli*, may be preferably used. As examples of host used in the step (iv), many cells are already known. Any cells may be used, and DH5 competent cell which has been prepared in accordance with the method described in Gene, vol. 96, pp. 23, 1990, may be preferably used. The random cloning in the step (v) may be carried out by methods known per se and the sequencing may be carried out in accordance with the method of Maxam-Gilbert or the dideoxy termination method. The step (vi) may be carried out in accordance with the method described in Molecular Cloning (written by Sambrook, J., Fritsch, E. F. and Maniatis, T., published by Cold Spring Harbor Laboratory Press in 1989).

As the following step, it is necessary to examine whether or not the DNA thus obtained codes right a protein including signal peptides. The examination requires:
(I) the conversion of the DNA sequence into the amino acid sequence in a possible frame,
(II) the preparation of hydrophobicity profile from the amino acid sequence conversed, followed by confirmation of the existence of a highly hydrophobic region just after the translation initiation codon (ATG)(membrane proteins have highly hydrophobic signal peptides on their N-termini), and then
(III) the confirmation that the DNA thus obtained covers complete or almost complete length of intact mRNA. These confirmation may be carried out after the step (vi) hereinbefore described, and effectively between the step (v) and the step (vi).

In the above examination, the step (II) may be carried out by analyzing using a computer making use of a hydro table of Kyte, J. and Doolittle, R.F., and an available the software, e.g. DIASIS (Hitachi Software Engineering Co., Ltd.). The step (III) may be carried out by Northern analysis.

Once the nucleotide sequences shown in SEQ ID Nos. 2 and 3 are determined, DNA of the present invention may be obtained by chemical synthesis, or by hybridization making use of a fragment of DNA of the present invention synthesized chemically, as a probe. Furthermore, DNA of the present invention may be obtained in a desired amount by transforming with a vector DNA inserted a DNA of the present invention into a proper host, followed by culturing the transformant.

The polypeptides of the present invention may be prepared by:
(1) isolating and purifying from an organism or a cultured cell,
(2) chemically synthesizing, or
(3) using a skill of biotechnology,
preferably, by the method described in (3).

Examples of expression system when preparing a polypeptide by using a skill of biotechnology is, for example, the expression system of bacteria, yeast, insect cell and mammalian cell.

For example, the expression in *E. coli* may be carried out by adding the initiation codon (ATG) to 5' end of a DNA encoding a mature protein, connecting the DNA thus obtained to the downstream of a proper promoter (e.g., *trp* promoter, *lac* promoter, λ*p*_{L} promoter, T7 promoter etc.), and then inserting it into a vector (e.g., pBR322, pUC18, pUC19 etc.) which functions in an *E. coli* strain to prepare an expression vector. Then, an *E. coli* strain (e.g., *E. coli* DH1 strain, *E. coli* JM109 strain, *E. coli* HB101 strain, etc.) which is transformed with the expression vector thus obtained may be cultured in a proper medium to obtain the desired polypeptide. When a signal peptide of bacteria (e.g., signal peptide of pel B) is utilized, the desired polypeptide may be also secreted in periplasm. Furthermore, a fusion protein with other polypeptide may be also produced easily.

Furthermore, the expression in a mammalian cell may be carried out, for example, by inserting the DNA shown in SEQ ID No. 3 into the downstream of a proper promoter (e.g., SV40 promoter, LTR promoter, metallothionein promoter etc.) in a proper vector (e.g., retrovirus vector, papilloma virus vector, vaccinia virus vector, SV40 vector, etc.) to obtain an expression vector, and transforming a proper mammalian cell (e.g., monkey COS-7 cell, Chinese hamster CHO cell, mouse L cell etc.) with the expression vector thus obtained, and then culturing the transformant in a proper medium to express a desired polypeptide on the cell membrane. The DNA encoding the nucleotide sequence shown in SEQ ID No.3 lacking transmembrane domain is inserted to the above vector. The appropriate mammalian cell is transfected or transformed by using the above DNA. And then the desired soluble polypeptide is secreted in the culture solution. The polypeptide thus obtained may be isolated and purified by conventional biochemical methods.

### Effects of the Invention

The polypeptide of the present invention is produced from an endothelial cell line of umbilical cord vein and may possess biological activities relating to adhesion of platelet, kinds of leukocytes (e.g. lymphocyte, neutrophil, eosinophilic leukocyte, basocyte and monocyte) and macrophage, relating to the function of immunity and relating to coagulation & fibrinogenolysis system.

The polypeptide of the present invention may be useful as itself or secretion type for the prevention of or in treatment of immune diseases caused by adhesion of platelet, kinds of leukocytes and macrophage and disease relating to thrombus formation (e.g. rheumatoid arthritic, allergy, arteriosclerosis, rejection after an internal organ plantation, myocardial infarction, brain infraction or repenfusion failure caused by it, DIC and septicemia etc.), or for screening test of the agent of the prevention of and/or in treatment of the above disease.

Further, polyclonal or monoclonal antibody against the polypeptide of the present invention can be used in the determination of the amount of the said polypeptide in organism, and thereby, may be utilized for the purpose of investigating the relationship between the said polypeptide and diseases, or for the purpose of diagnosing diseases, and the like. Polyclonal and monoclonal antibody thereof may be prepared by conventional methods by using the said polypeptide or the fragment thereof as an antigen.

The DNA of the present invention may be utilized as an important and essential template in preparing the polypeptide of the present invention which is expected to possess various use or for diagnosis of and in the treatment of gene diseases (the treatment of gene defect disease and the treatment by inhibiting expression of the polypeptide by antisense DNA (RNA), and the like). Further, genomic DNA may be isolated by using the DNA of the present invention as a probe. Similarly, it is possible to isolate genes having high homology to the DNA of the present invention in human or those of other species.

### Application for Pharmaceuticals

For the purpose of the present invention, the polypeptide of the present invention may be normally administered systemically or partially, usually by oral or parenteral administration as a solution, preferably orally, intravenously or intraventricularly.

The doses to be administered are determined depending upon age, body weight, symptom, the desired therapeutic effect, the route of administration, and the duration of the treatment etc. In the human adult, the doses per person per dose are generally between 100 µg and 100 mg, by oral administration, up to several times per day, and between 10 µg and 100 mg, by parenteral administration up to several times per day.

As mentioned above, the doses to be used depend upon various conditions. Therefore, there are cases in which doses lower than or greater than the ranges specified above may be used.

Administration of the compounds of the present invention, may be as solid compositions, liquid compositions or other compositions for oral administration, as injections, liniments or suppositories etc. for parenteral administration.

Solid compositions for oral administration include compressed tablets, pills, capsules, dispersible powders, granules. Capsules include soft capsules and hard capsules.

In such compositions, one or more of the active compound(s) is or are admixed with at least one inert diluent (such as lactose, mannitol, glucose, hydroxypropyl cellulose, microcrystalline cellulose, starch, polyvinylpyrrolidone, magnesium metasilicate aluminate, etc.). The compositions may also comprise, as is normal practice, additional substances other than inert diluents: e.g. lubricating agents (such as magnesium stearate etc.), disintegrating agents (such as cellulose calcium glycolate, etc.), stabilizing agents (such as human serum albumin, lactose etc.), and assisting agents for dissolving (such as arginine, asparaginic acid etc.).

The tablets or pills may, if desired, be coated with a film of gastric or enteric material (such as sugar, gelatin, hydroxypropyl cellulose or hydroxypropylmethyl cellulose phthalate, etc.), or be coated with more than two films. And further, coating may include containment within capsules of absorbable materials such as gelatin.

Liquid compositions for oral administration include pharmaceutically-acceptable emulsions, solutions, syrups and elixirs. In such compositions, one or more of the active compound(s) is or are contained in inert diluent(s) commonly used in the art (purified water, ethanol etc.). Besides inert diluents, such compositions may also comprise adjuvants (such as wetting agents, suspending agents, etc.), sweetening agents, flavouring agents, perfuming agents, and preserving agents.

Other compositions for oral administration included spray compositions which may be prepared by known methods and which comprise one or more of the active compound(s). Spray compositions may comprise additional substances other than inert diluents: e.g. stabilizing agents (sodium sulfite etc.), isotonic buffer (sodium chloride, sodium citrate, citric acid, etc.). For preparation of such spray compositions, for example, the method described in the United States Patent No. 2,868,691 or 3,095,355 (herein incorporated in their entireties by reference) may be used.

Injections for parenteral administration include sterile aqueous or non-aqueous solutions, suspensions and emulsions. In such compositions, one or more active compound(s) is or are admixed with at least one inert aqueous diluent(s) (distilled water for injection, physiological salt solution, etc.) or inert non-aqueous diluents(s)(propylene glycol, polyethylene glycol, olive oil, ethanol, POLYSOLBATE 80 TM, etc.).

Injections may comprise additional other than inert diluents: e.g. preserving agents, wetting agents, emulsifying agents, dispersing agents, stabilizing agent (such as human serum albumin, lactose, etc.), and assisting agents such as assisting agents for dissolving (arginine, asparaginic acid, etc.).

They may be sterilized for example, by filtration through a bacteria-retaining filter, by incorporation of sterilizing agents in the compositions or by irradiation. They may also be manufactured in the form of sterile solid compositions, for example, by freeze-drying, and which can be dissolved in sterile water or some other sterile diluents for injection immediately before used.

Other compositions for parenteral administration include liquids for external use, and endermic liniments (ointment, etc.), suppositories for rectal administration and pessaries which comprise one or more of the active compound(s) and may be prepared by known methods.

### Examples

The following examples are illustrated, but not limit, the present invention.

### Example 1 : Construction of plasmid vector for use in the preparation of cDNA library

A vector, called pcD-SRα296, constructed by Takebe et al. vector (Mol. Cell. Biol., vol. 8, p 966, 1988) is an excellent expression vector for use in animal cells, which contains a promoter system (SRα) consisting of SV40 early promoter, R region of LTR of HTLV-1 and a part of U5 sequence. This vector, however, has disadvantages in that (1) it has only one insert-cloning site, namely an EcoRI site, and (2) its recovery yield from *E. Coli* is small because of the use of pBR322 vector as its basic structure.

It consequence, an attempt was made to modify the pcD-SRα296 vector in the following manner, with the aim of constructing a modified vector containing multiple insert-cloning sites and having a basic structure derived from pUC19 which is effective in recovering the resulting modified vector in high yield from *E.Coli*.

The pcD-SRα296 vector (provided by Dr. Takebe at National Institute of Health) was digested with *Sal*I and the resulting digest was subjected to agarose gel electrophoresis to separate and recover a 1.7 kbp fragment containing the SRα promoter, subsequently smooth-ending the thus obtained fragment by Klenow treatment.

Separately from this, pUC19 vector was digested with *Nde*I and *Hind*III and the resulting digest was subjected to agarose gel electrophoresis to separate and recover a 2.4 kbp fragment containing Amp^{r} and pUCori regions, subsequently smooth-ending the thus obtained fragment by Klenow treatment and thereafter removing the 5'-end phosphoric acid group by BAP (bacterial alkaline phosphatase) treatment.

The thus prepared 1.7 kbp fragment containing the SRα promoter was ligated with the pUCori-containing 2.4 kbp fragment to make them into a circular form, thereby effecting construction of a new-vector. A *Pst*I-*Kpn*I fragment was removed from the thus obtained vector and replaced with the following synthetic polylinker.

### SEQ ID No. 5

SacI SalI BglII EcoRI NdeI SmaI NotI SpeI
5' GGAGCTCGTCGACAGATCTGAATTCCATATGCCCGGGCGGCCGCACTAGTGGTAC 3'
3' ACGTCCTCGAGCAGCTGTCTAGACTTAAGGTATACGGGCCCGCCGGCGTGATCAC 5'

### SEQ ID No. 6

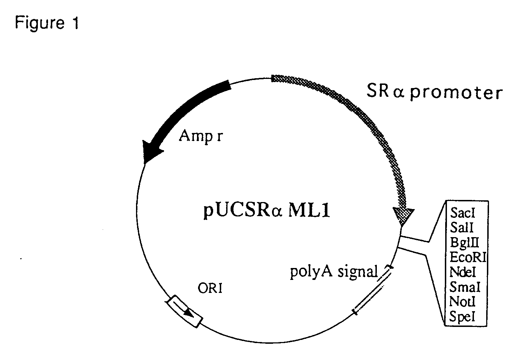

The plasmid vector thus constructed (about 3.9 kbp, see Fig. 1) was named pUCSRαML-1.

The pUCSRαML-1 has the following characteristic properties as a multi-purpose plasmid vector.
1. Plasmid yield per cultured cells is high.
2. pUCSRαML1 can express directly and use for expression cloning by transfection to COS cell.

### Example 2 : Isolation and purification of mRNA

By using total of 3 x 10⁷ cells of an endothelial cell line of human umbilical cord vein, mRNA were isolated in accordance with the method of Okayama, H. et al. (Methods in Enzymology, vol. 154, pp. 3, 1987).

That is, the stimulated cells were solubilized with a 5.5 M GTC solution (5.5 M guanidine thiocyanate, 25 mM sodium citrate, 0.5% sodium lauryl sarcosine), and the resulting cell lysate was laid on a cushion of a cesium trifluoroacetate (CsTFA) solution having a density of 1.51 and centrifuged (120,000 x g, 20 hours) to recover 1.26 mg of total RNA in the resulting pellet. Thereafter, the RNA sample was passed twice through an oligo(dT)-cellulose column to purify and recover 46 µg of poly(A)⁺RNA.

### Example 3 : Preparation of cDNA library

A cDNA library was prepared in accordance with the method of Gubler & Hoffman (Gene, vol. 25, pp. 263, 1983) with a slight modification.

A first strand was synthesized from the poly(A)⁺RNA (5 µg) prepared in Example 2, using a reverse transcriptase and an oligo(dT) primer having a *Not*I site. After synthesizing a second strand and carrying out *Sal*I adaptor ligation and *Not*I digestion, the adaptor and the primer were removed by gel filtration column chromatography using a column packed with Sephacryl S-500HR (available from Pharmacia), thereby recovering a fraction containing 820 ng of cDNA.

The above cDNA synthesizing step was effected making use of a kit (Super Script System, available from BRL).

Separately from this, a vector was prepared by subjecting the pVfCS-1 obtained in Example 1 to complete digestion with *Not*I, digesting the product further with *Sal*I, subjecting the resulting digest to 0.8% agarose gel electrophoresis to cut out a band of interest and then purifying the vector of interest making use of a kit for glass powder method use (GENECLEAN II, available from BIO 101).

After subjecting the thus prepared cDNA and vector to ligation, the resulting product was transfected into DH5 competent cells which have been prepared in accordance with the method of Inoue, H. et al. (Gene, vol. 96, pp. 23, 1990). As the results, a cDNA library containing 6 x 10⁵ independent clones with an average length of 1.5 kb was obtained.

### Example 4 : Cloning and Sequencing

Using the cDNA library prepared in Example 3, clones were plated on LB-broth agar containing anpicillin with a density of 300 colonies/dish having a diameter of 10 cm. Cloning was carried out by picking up the colonies at random. Each of the colonies was cultured overnight in 3 ml of LB-broth. A 200 µl portion of the resulting culture was mixed with dimethylsulfoxide (DMSO, final concentration of 7%) and stored at -80°C, and the remaining portion of the culture was used for the isolation of plasmid. The purification of plasmid was carried out by the usual way.

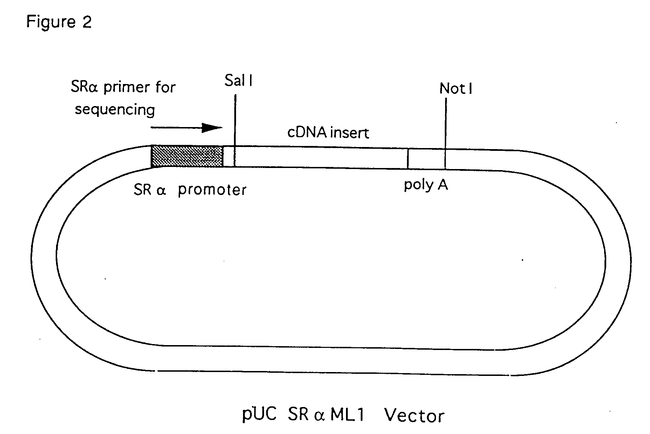

Since the plasmid has a structure shown in Fig. 2, its nucleotide sequence can be read from the 5' end of the cloned cDNA when sequencing is carried out using SRα primer.

DNA sequencing was carried out in accordance with a cycle sequence method based on the dideoxy termination method of Sanger, F. et al., using a fluorescence dye terminator of ABI (Applied Biosystems Inc.). Reading of the sequence was carried out using a DNA sequencer of ABI (Model 373A).

In this way, a nucleotide sequence having a length of about 300 bases from 5' end of each cDNA was obtained.

### Example 5 : Analysis of partial sequence data

Using the FASTA program of Lipman, D. J. and Pearson, W. R., the nucleotide sequence obtained in Example 4 was searched for its homology with every nucleotide sequence contained in known data bases (GenBank and EMBL). As the results, a clone having an unknown sequence was identified. The unknown nucleotide sequence was converted into amino acid sequences in possible three frames.

Next, hydrophobic and hydrophilic profiles of the amino acid sequences deduced from their corresponding three frames were analyzed graphically using a computer making use of a hydro table of Kyte, J. and Doolittle, R. F. In this instance, DNASIS (Hitachi Software Engineering Co., Ltd.) was used as a computer software.

It is known that most of cytokines and growth factors, as well as a number of membrane proteins, have signal peptides on their N-termini. Signal peptide is highly hydrophobic region immediately downstream of the translation initiation amino acid Met. In a nucleotide sequence, it is located just after the initiation codon (ATG) of its coding region. In consequence, a cDNA having a putative signal peptide-containing protein was screened on the basis of data on the open reading frame of the clone having unknown sequence and on the hydrophobic and hydrophilic properties of the amino acid sequence. As the result, a clone of interest (clone No. ET141) was found which showed signal peptide-specific nature in one of its three possible amino acid sequence frames. (See Figure 3 which shows a hydrophobicity profile of the total amino acid sequence. In the figure, the "+" side is a highly hydrophobic region and the "-" side is a region having weak hydrophobicity.)

It is possible however that the cloned cDNA does not cover complete length of mRNA. If it does not cover the complete length, it is less possible that the clone contains the N-terminal amino acid sequence moiety.

In consequence, Northern analysis was carried out in order to determine if the clone ET141 has complete length or not. That is, poly(A)⁺RNA which has been extracted and purified from a vascular endothelial cell line was subjected to electrophoresis and then blotting on nylon membrane.

When hybridization was carried out using a ET141 cDNA insert as a probe, a single band was found at a position corresponding to about 1100 bp. Since the ET141 cDNA insert had a size of about 1200 bp, it was confirmed that the ET141 clone was a almost complete length cDNA.

### Example 6 : Determination of complete sequence of the cDNA and open reading frame

The complete length cDNA sequence was determined by means of random sequencing in accordance with the method described in Molecular Cloning (Sambrook, J., Fritsch, E. F. and Maniatis, T., Cold Spring Harbor Laboratory Press, 1989).

Plasmid was recovered from the ET141 clone to isolate and purify a cDNA insert. The insert thus purified was subjected to ligation and fragmentation, followed by smooth-ending of both termini of the resulting DNA fragment with T4 polymerase, thereby recovering a DNA fragment of about 400 bp in length by agarose gel electrophoresis. The thus obtained DNA fragment was cloned into a *Sma*I site of a plasmid vector BLUESCRIPT II (available from Stratagene) and then transfected into an *E. coli* strain. A total of 90 colonies were picked up at random to prepare 90 corresponding plasmid DNA samples (every of them contained a ET141 cDNA fragment as an insert) which were subsequently subjected to DNA sequencing. DNA sequencing and sequence reading were carried out in the same manner as the procedure described in Example 4. Sequence data of the ET141 cDNA fragment were arranged into a continued sequence making use of a DNA sequence connection program of DNASIS, thereby obtaining a base sequence shown in SEQ ID No. 3. An open reading frame was determined based on the complete length cDNA sequence data to deduce corresponding amino acid sequence, with the results shown in SEQ ID No. 1. The amino acid sequence which is located from the amino acid at the 1st position to amino acid at the 21th position is theoretically signal peptide. The amino acid sequence which is located from the amino acid at the 119th position to the amino acid at the 146th position was thought to be cell transmembrane region by using hydrophobicity profile (shown in Figure 3). The region which consists of three amino acids which is located from the amino acid at the 96th position is probably shown a region which N-linked glycochain binds.

SEQUENCE LISTING

(1) GENERAL INFORMATION:

(i) APPLICANT:
(A) NAME: ONO PHARMACEUTICAL CO, LTD.
(B) STREET: 1-5, Doshomachi, 2-chome
(C) CITY: Chuo-ku, Osaka-shi
(D) STATE: Osaka
(E) COUNTRY: Japan
(F) POSTAL CODE (ZIP): 541
(G) TELEPHONE: (06)222-5551
(H) TELEFAX: (06)222-5706

(ii) TITLE OF INVENTION: A NOVEL POLYPEPTIDE AND DNAS ENCODING IT

(iii) NUMBER OF SEQUENCES: 6

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(2) INFORMATION FOR SEQ ID NO:1:

(i) SEQUENCE CHARACTERISTICS:
(A) LENGTH: 205 amino acids
(B) TYPE: amino acid
(D) TOPOLOGY: linear

(ii) MOLECULE TYPE: protein

(xi) SEQUENCE DESCRIPTION: SEQ ID NO:1:

Met Gly Thr Ala Gly Ala Met Gln Leu Cys Trp Val Ile Leu Gly Phe
-24             -20                 -15                 -10

Leu Leu Phe Arg Gly His Asn Ser Gln Pro Thr Met Thr Gln Thr Ser
            -5                  1               5

Ser Ser Gln Gly Gly Leu Gly Gly Leu Ser Leu Thr Thr Glu Pro Val
    10                  15                  20

Ser Ser Asn Pro Gly Tyr Ile Pro Ser Ser Glu Ala Asn Arg Pro Ser
25                  30                  35                  40

His Leu Ser Ser Thr Gly Thr Pro Gly Ala Gly Val Pro Ser Ser Gly
                45                  50                  55

Arg Asp Gly Gly Thr Ser Arg Asp Thr Phe Gln Thr Val Pro Pro Asn
            60                  65                  70

Ser Thr Thr Met Ser Leu Ser Met Arg Glu Asp Ala Thr Ile Leu Pro
        75                  80                  85

Ser Pro Thr Ser Glu Thr Val Leu Thr Val Ala Ala Phe Gly Val Ile
    90                  95                  100

Ser Phe Ile Val Ile Leu Val Val Val Val Ile Ile Leu Val Gly Val
105                 110                 115                 120

Val Ser Leu Arg Phe Lys Cys Arg Lys Ser Lys Glu Ser Glu Asp Pro
                125                 130                 135

Gln Lys Pro Gly Ser Ser Gly Leu Ser Glu Ser Cys Ser Thr Ala Asn
            140                 145                 150

Gly Glu Lys Asp Ser Ile Thr Leu Ile Ser Met Lys Asn Ile Asn Met
        155                 160                 165

Asn Asn Gly Lys Gln Ser Leu Ser Ala Glu Lys Val Leu
    170                 175                 180

(2) INFORMATION FOR SEQ ID NO:2:

     (i) SEQUENCE CHARACTERISTICS:
          (A) LENGTH: 618 base pairs
          (B) TYPE: nucleic acid
          (C) STRANDEDNESS: single
          (D) TOPOLOGY: linear

    (ii) MOLECULE TYPE: cDNA to mRNA

    (xi) SEQUENCE DESCRIPTION: SEQ ID NO:2:

ATGGGCACCG CAGGAGCCAT GCAGCTGTGC TGGGTGATCC TGGGCTTCCT CCTGTTCCGA      60

GGCCACAACT CCCAGCCCAC AATGACCCAG ACCTCTAGCT CTCAGGGAGG CCTTGGCGGT     120

CTAAGTCTGA CCACAGAGCC AGTTTCTTCC AACCCAGGAT ACATCCCTTC CTCAGAGGCT     180

AACAGGCCAA GCCATCTGTC CAGCACTGGT ACCCCAGGCG CAGGTGTCCC CAGCAGTGGA     240

AGAGACGGAG GCACAAGCAG AGACACATTT CAAACTGTTC CCCCCAATTC AACCACCATG     300

AGCCTGAGCA TGAGGGAAGA TGCGACCATC CTGCCCAGCC CCACGTCAGA GACTGTGCTC     360

ACTGTGGCTG CATTTGGTGT TATCAGCTTC ATTGTCATCC TGGTGGTTGT GGTGATCATC     420

CTAGTTGGTG TGGTCAGCCT GAGGTTCAAG TGTCGGAAGA GCAAGGAGTC TGAAGATCCC     480

CAGAAACCTG GGAGTTCAGG GCTGTCTGAA AGCTGCTCCA CAGCCAATGG AGAGAAAGAC     540

AGCATCACCC TTATCTCCAT GAAGAACATC AACATGAATA ATGGCAAACA AAGTCTCTCA     600

GCAGAGAAGG TTCTTTAA                                                   618

(2) INFORMATION FOR SEQ ID NO:3:

(i) SEQUENCE CHARACTERISTICS:
(A) LENGTH: 983 base pairs
(B) TYPE: nucleic acid
(C) STRANDEDNESS: single
(D) TOPOLOGY: linear

(ii) MOLECULE TYPE: cDNA to mRNA

(xi) SEQUENCE DESCRIPTION: SEQ ID NO:3:

GCCTGCCCGC CACATACCCA GCTGACATGG GCACCGCAGG AGCCATGCAG CTGTGCTGGG 60

TGATCCTGGG CTTCCTCCTG TTCCGAGGCC ACAACTCCCA GCCCACAATG ACCCAGACCT 120

CTAGCTCTCA GGGAGGCCTT GGCGGTCTAA GTCTGACCAC AGAGCCAGTT TCTTCCAACC 180

CAGGATACAT CCCTTCCTCA GAGGCTAACA GGCCAAGCCA TCTGTCCAGC ACTGGTACCC 240

CAGGCGCAGG TGTCCCCAGC AGTGGAAGAG ACGGAGGCAC AAGCAGAGAC ACATTTCAAA 300

CTGTTCCCCC CAATTCAACC ACCATGAGCC TGAGCATGAG GGAAGATGCG ACCATCCTGC 360

CCAGCCCCAC GTCAGAGACT GTGCTCACTG TGGCTGCATT TGGTGTTATC AGCTTCATTG 420

TCATCCTGGT GGTTGTGGTG ATCATCCTAG TTGGTGTGGT CAGCCTGAGG TTCAAGTGTC 480

GGAAGAGCAA GGAGTCTGAA GATCCCCAGA AACCTGGGAG TTCAGGGCTG TCTGAAAGCT 540

GCTCCACAGC CAATGGAGAG AAAGACAGCA TCACCCTTAT CTCCATGAAG AACATCAACA 600

TGAATAATGG CAAACAAAGT CTCTCAGCAG AGAAGGTTCT TTAAAAGCAA CTTTGGGTCC 660

CCATGAGTCC AAGGATGATG CAGCTGCCCT GTGACTACAA GGAGGAAGAG ATGGAATTAG 720

TAGAGGCAAT GAACCACATG TAAATTATTT TATTGTTTCA TGTCTGCTTC TAGATCTAAA 780

GGACACTAGC ATTGCCCCAG ATCTGGGAGC AAGCTACCAA CAGGGGAGAC TCTTTCCTGT 840

ATGGACAGCT GCTGTGGAAA TACTGCCTGC TTCTCCCACC TCCTCAGAGC CACAGGAAAG 900

AGGAGGTGAC AGAGAGAGAG CAAGGAAAGT GATGAGGTGG ATTGATACTT TCTACTTTGC 960

ATTAAAATTA TTTTCTAGCC TGC 983

(2) INFORMATION FOR SEQ ID NO:4:

(i) SEQUENCE CHARACTERISTICS:
(A) LENGTH: 983 base pairs
(B) TYPE: nucleic acid
(C) STRANDEDNESS: single
(D) TOPOLOGY: linear

(ii) MOLECULE TYPE: cDNA to mRNA

(vi) ORIGINAL SOURCE:
(A) ORGANISM: Homo sapiens

(H) CELL LINE: endothelial cell line of umbilical cord vein

(ix) FEATURE:
(A) NAME/KEY: CDS
(B) LOCATION: 27..644
(C) IDENTIFICATION METHOD: by similarity to some other pattern

(ix) FEATURE:
(A) NAME/KEY: sig_peptide
(B) LOCATION: 27..98
(C) IDENTIFICATION METHOD: by similarity with known sequence or
to an established consensus

(ix) FEATURE:
(A) NAME/KEY: misc_feature
(B) LOCATION: 381..464
(C) IDENTIFICATION METHOD: by similarity with known sequence or
to an established consensus

(ix) FEATURE:
(A) NAME/KEY: mat_peptide
(B) LOCATION: 99..641
(C) IDENTIFICATION METHOD: by similarity with known sequence or
to an established consensus

(ix) FEATURE:
(A) NAME/KEY: misc_feature
(B) LOCATION: 312..320
(C) IDENTIFICATION METHOD: by similarity with known sequence or
to an established consensus

(xi) SEQUENCE DESCRIPTION: SEQ ID NO:4:

GCCTGCCCGC CACATACCCA GCTGAC ATG GGC ACC GCA GGA GCC ATG CAG CTG 53
Met Gly Thr Ala Gly Ala Met Gln Leu
-24 -20

TGC TGG GTG ATC CTG GGC TTC CTC CTG TTC CGA GGC CAC AAC TCC CAG 101
Cys Trp Val Ile Leu Gly Phe Leu Leu Phe Arg Gly His Asn Ser Gln
-15 -10 -5 1

CCC ACA ATG ACC CAG ACC TCT AGC TCT CAG GGA GGC CTT GGC GGT CTA 149
Pro Thr Met Thr Gln Thr Ser Ser Ser Gln Gly Gly Leu Gly Gly Leu
                5                   10                  15

AGT CTG ACC ACA GAG CCA GTT TCT TCC AAC CCA GGA TAC ATC CCT TCC 197
Ser Leu Thr Thr Glu Pro Val Ser Ser Asn Pro Gly Tyr Ile Pro Ser
            20                  25                  30

TCA GAG GCT AAC AGG CCA AGC CAT CTG TCC AGC ACT GGT ACC CCA GGC 245
Ser Glu Ala Asn Arg Pro Ser His Leu Ser Ser Thr Gly Thr Pro Gly
        35                  40                  45

GCA GGT GTC CCC AGC AGT GGA AGA GAC GGA GGC ACA AGC AGA GAC ACA 293
Ala Gly Val Pro Ser Ser Gly Arg Asp Gly Gly Thr Ser Arg Asp Thr
50 55 60 65

TTT CAA ACT GTT CCC CCC AAT TCA ACC ACC ATG AGC CTG AGC ATG AGG 341

Phe Gln Thr Val Pro Pro Asn Ser Thr Thr Met Ser Leu Ser Met Arg
                70                  75                  80

GAA GAT GCG ACC ATC CTG CCC AGC CCC ACG TCA GAG ACT GTG CTC ACT     389
Glu Asp Ala Thr Ile Leu Pro Ser Pro Thr Ser Glu Thr Val Leu Thr
            85                  90                  95

GTG GCT GCA TTT GGT GTT ATC AGC TTC ATT GTC ATC CTG GTG GTT GTG     437
Val Ala Ala Phe Gly Val Ile Ser Phe Ile Val Ile Leu Val Val Val
        100                 105                 110

GTG ATC ATC CTA GTT GGT GTG GTC AGC CTG AGG TTC AAG TGT CGG AAG     485
Val Ile Ile Leu Val Gly Val Val Ser Leu Arg Phe Lys Cys Arg Lys
    115                 120                 125

AGC AAG GAG TCT GAA GAT CCC CAG AAA CCT GGG AGT TCA GGG CTG TCT     533
Ser Lys Glu Ser Glu Asp Pro Gln Lys Pro Gly Ser Ser Gly Leu Ser
130                 135                 140                 145

GAA AGC TGC TCC ACA GCC AAT GGA GAG AAA GAC AGC ATC ACC CTT ATC     581
Glu Ser Cys Ser Thr Ala Asn Gly Glu Lys Asp Ser Ile Thr Leu Ile
                150                 155                 160

TCC ATG AAG AAC ATC AAC ATG AAT AAT GGC AAA CAA AGT CTC TCA GCA     629
Ser Met Lys Asn Ile Asn Met Asn Asn Gly Lys Gln Ser Leu Ser Ala
            165                 170                 175

GAG AAG GTT CTT TAA AAGCAACTTT GGGTCCCCAT GAGTCCAAGG ATGATGCAGC     684
Glu Lys Val Leu  *
        180

TGCCCTGTGA CTACAAGGAG GAAGAGATGG AATTAGTAGA GGCAATGAAC CACATGTAAA     744

TTATTTTATT GTTTCATGTC TGCTTCTAGA TCTAAAGGAC ACTAGCATTG CCCCAGATCT     804

GGGAGCAAGC TACCAACAGG GGAGACTCTT TCCTGTATGG ACAGCTGCTG TGGAAATACT     864

GCCTGCTTCT CCCACCTCCT CAGAGCCACA GGAAAGAGGA GGTGACAGAG AGAGAGCAAG     924

GAAAGTGATG AGGTGGATTG ATACTTTCTA CTTTGCATTA AAATTATTTT CTAGCCTGC     983

(2) INFORMATION FOR SEQ ID NO:5:

     (i) SEQUENCE CHARACTERISTICS:
          (A) LENGTH: 56 base pairs
          (B) TYPE: nucleic acid
          (C) STRANDEDNESS: double
          (D) TOPOLOGY: linear

    (ii) MOLECULE TYPE: DNA (synthetic)

    (xi) SEQUENCE DESCRIPTION: SEQ ID NO:5:

GGAGCTCGTC GACAGATCTG AATTCCATAT GCCCGGGGCG GCCGCACTAG TGGTAC     56

(2) INFORMATION FOR SEQ ID NO:6:

(i) SEQUENCE CHARACTERISTICS:
(A) LENGTH: 56 base pairs
(B) TYPE: nucleic acid
(C) STRANDEDNESS: double
(D) TOPOLOGY: linear

(ii) MOLECULE TYPE: DNA (synthetic)

(xi) SEQUENCE DESCRIPTION: SEQ ID NO:6:

CACTAGTGCG GCCGCCCCGG GCATATGGAA TTCAGATCTG TCGACGAGCT CCTGCA 56

### Brief description of the drawings

Figure 1 shows the construction of plasmid vector, pUCSRαML-1.

Figure 2 shows the construction of a recombinant DNA into which cDNA derived from endothelial cell line of human umbilical cord vein is inserted.

Figure 3 shows hydrophobicity profile of the polypeptide of the present invention.

Figure 4 shows amino acid constitution of the polypeptide of the present invention.

## Claims

1. A polypeptide having the amino acid sequence shown in SEQ ID No. 1 in substantially purified form, a homologue thereof or a fragment of the sequence or homologue of a fragment.

2. A polypeptide according to claim 1 having the amino acid sequence shown in SEQ ID No. 1.

3. DNA encoding a polypeptide according to claim 1.

4. DNA according to claim 3 having the nucleotide sequence shown in SEQ ID No. 2 or a fragment thereof capable of selectively hybridizing to SEQ ID No. 2.

5. DNA according to claim 3 having the nucleotide sequence shown in SEQ ID No. 3 or a fragment thereof capable of selectively hybridizing to SEQ ID No. 3.

6. A replication and expression vector comprising DNA according to any one of claims 3 to 5.

7. Host cells transformed or transfected with a replication and expression vector according to claim 6.

8. A method of producing a polypeptide which comprises culturing host cells according to claim 7 under conditions effective to express a polypeptide according to claim 1 or 2.

9. A monoclonal or polyclonal antibody to a polypeptide according to claim 1 or 2.

10. A pharmaceutical composition containing a polypeptide according to claims 1 or 2 or an antibody according to claim 9 in association with a pharmaceutically acceptable diluent and/or carrier.
